# EUROPEAN PATENT APPLICATION

(11) **EP 4 371 573 A1**
(43) Date of publication of application: **22.05.2024**
(21) Application number: 22842038.6
(22) Date of filing: 07.07.2022
(51) Int. Cl.: A61K 39/395, A61K 45/00, A61P 1/04, A61P 43/00, C07K 16/24, C07K 19/00, C12N 15/13, C12P 21/08

(54) **MEDICINAL COMPOSITION FOR TREATING INFLAMMATORY BOWEL DISEASE**

(30) Priority: 13.07.2021 JP 2021115458
(71) Applicant: National University Corporation Tokai National Higher Education and Research System, Nagoya-shi, Aichi 464-8601 (JP); Mabprotein Co.,Ltd., Izumo-shi, Shimane, 693-8501 (JP)
(72) Inventor: MAEDA, Keiko, Nagoya-shi, Aichi 464-8601 (JP); FUJISHIRO, Mitsuhiro, Nagoya-shi, Aichi 464-8601 (JP); IKEGAMI, Shuji, Nagoya-shi, Aichi 464-8601 (JP); URANO, Takeshi, Izumo-shi, Shimane 693-8501 (JP)
(74) Representative: Schön, Christoph
(86) International application number: PCT/JP2022/027035
(87) International publication number: WO 2023/286694

(57) **Abstract**

About 5 million people worldwide are said to suffer from inflammatory bowel disease, including ulcerative colitis and Crohn's disease, and development of a therapeutic agent is desired. In particular, the development of an effective therapeutic agent for patients who are resistant to existing therapeutic agents is a problem of concern. A neutralizing antibody recognizing only active IL-18 is found to have an effect of ameliorating enteritis. Furthermore, the use of an anti-TNF-α antibody in combination therewith is found to have a remarkable effect of ameliorating enteritis. A pharmaceutical composition containing, as active ingredients, an antibody recognizing active IL-18, i.e., an antibody recognizing a neoepitope, and a TNF-α inhibitor has a significant effect of ameliorating enteritis, and thus serves as a therapeutic agent for inflammatory bowel disease.

## Description

### Technical Field

The present invention relates to a pharmaceutical composition for treating inflammatory bowel disease, the pharmaceutical composition containing an antibody recognizing active IL-18 as an active ingredient.

### Background Art

Inflammatory bowel disease (IBD) includes ulcerative colitis and Crohn's disease as main forms, both of which are designated as intractable diseases. IBD is a disease that causes inflammation in the mucosa of the gastrointestinal tract, mainly of the small intestine and the large intestine, with symptoms such as diarrhea, hematochezia, abdominal pain, and fever. Ulcerative colitis and Crohn's disease being main forms are diseases of unknown cause, which are estimated to affect about 300,000 people nationwide and about 5 million worldwide, with a rapid increase in the number of patients, especially the young.

For the treatment of IBD, 5-aminosalicylic acid preparations, immunomodulators such as azathioprine, and corticosteroids are used, and biologics are introduced in the case of exhibiting treatment resistance to these drugs. While the emergence of an anti-TNF-α antibody preparation, one of the biologics, has revolutionized treatment (Non Patent Literature 1), treatment resistance has been observed in about half of the patients (Non Patent Literatures 2 and 3), and there is an urgent need to establish a novel therapy tailored to a pathological condition.

In addition to dysbiosis and disruption of mucosal immunity, the importance of the inflammasome pathway in relation to both has been recognized in the pathological condition of IBD. A plurality of inflammasome-related genes has been identified as disease-susceptibility genes for IBD, and functional abnormalities thereof have been reported to induce dysbiosis, activate type 1 helper T (Th1) cells and Th2 cells through the production of active IL-18, and exacerbate enteritis.

The present inventors have found that IL-18, an inflammasome-related cytokine, is highly expressed in the blood and intestinal epithelium and stromal tissues of patients with IBD who are resistant to treatment with anti-TNF-α antibody and anti-IL-12/23 antibody preparations. An anti-active IL-18 neutralizing antibody, which directly suppresses active IL-18 produced by inflammasome activation, has a different pathway from the existing TNF-α and IL-12/23 pathways, and therefore, is expected to be effective as a novel therapeutic agent for intractable cases. Besides, since IL-18 gene-deficient mice do not exhibit severe symptoms, IL-18 suppression is less likely to cause serious side effects and can be a promising drug candidate. Hence, an investigation was conducted to determine the feasibility of treating inflammatory bowel disease using an anti-IL-18 antibody.

IL-18 is a cytokine of the IL-1β family, which is expressed mainly in macrophages but also in various cells such as dendritic cells, epithelial cells, and keratinocytes. An IL-18 receptor is expressed in various cells such as B cells, neutrophils, macrophages, vascular endothelial cells, smooth muscle cells, as well as NK cells, NKT cells, and CD4 T cells. In intestinal tissues, IL-18 is produced as a precursor from macrophages, dendritic cells, and intestinal epithelial cells, cleaved by a caspase produced by inflammasome, and secreted extracellularly as active IL-18. An anti-active IL-18 neutralizing antibody, which directly suppresses active IL-18 produced by inflammasome activation, has a different mechanism from the existing TNF-α and IL-12/23 pathways, and therefore, is expected to be effective as a novel therapeutic agent for intractable cases. However, it has already been disclosed that one that suppresses IL-18, although not with an active IL-18 neutralizing antibody, can be used as a therapeutic agent for inflammatory bowel disease (Patent Literatures 1 to 10).

### Citation List

### Patent Literature

Patent Literature 1: International Publication No. WO 2015/032932
Patent Literature 2: International Publication No. WO 2014/186728
Patent Literature 3: International Publication No. WO 2014/037899
Patent Literature 4: International Publication No. WO 2001/058956
Patent Literature 5: International Publication No. WO 2005/047307
Patent Literature 6: International Publication No. WO 2012/085015
Patent Literature 7: International Publication No. WO 2010/048183
Patent Literature 8: International Publication No. WO 2007/137984
Patent Literature 9: International Publication No. WO 2001/062285
Patent Literature 10: International Publication No. WO 2000/056771
Patent Literature 11: International Publication No. WO 2020/116423
Patent Literature 12: International Publication No. WO 2004/092219

### Non Patent Literature

Non Patent Literature 1: Nielsen and Ainsworth, 2013, N Engl J Med, Vol.369(8), p.754-762
Non Patent Literature 2: Gisbert et al., 2015, Aliment. Pharmacol. Ther. Vol.41(7), p.613-23
Non Patent Literature 3: Singh et al., 2018, Aliment. Pharmacol. Ther. Vol. 47(2), p.162-175
Non Patent Literature 4: Arimori, T., et al., Structure, 2017, Vol. 25, pp.1611-1622
Non Patent Literature 5: Hezareh et al., J. Virol. 2001, Vol. 75, pp.12161-12168
Non Patent Literature 6: Oganesyan et al., Acta Cryst., 2008, D64, pp.700-704
Non Patent Literature 7: Tao and Morrison, J. Immunol., 1989, Vol. 143, pp.2595-2601
Non Patent Literature 8: Shields et al., J. Biol. Chem., 2001, Vol. 276, pp.6591-6604
Non Patent Literature 9: Stefan Wirtz et al., Nature Protocols, 2017, Vol.12, pp.1295-1309
Non Patent Literature 10: Molgora et al., Nature, 2017, Vol. 551(7678), pp. 110-114

### Summary of Invention

### Technical Problem

Patent Literature 1 to 10 disclose Crohn's disease or ulcerative colitis as one of inflammatory diseases to be treated. However, no single literature has actually shown its efficacy. Although several companies currently conduct and prepare clinical trials, there are no anti-IL-18 antibody drugs that have been used at a practical level. Clinical trials of anti-IL-18 antibody medicaments have been conducted for sarcoidosis (NCT04064242, Phase II, Patent Literature 3), atopic dermatitis (NCT04836858, Phase II, Patent Literature 3), transplant rejection (NCT02723786, Phase II, Patent Literature 8), type 2 diabetes mellitus (NCT01648153, Phase II, Patent Literature 8), Behcet's disease (NCT03522662, Phase II, Patent Literature 8), Crohn's disease (NCT03681067, Phase II, Patent Literature 8), inflammatory bowel disease (NCT01035645, Phase I, Patent Literature 8), adult-onset Still's disease (NCT04752371, Phase I, Patent Literature 6), and multiple myeloma (NCT04671251, Phase I, Patent Literature 6) as target diseases. However, no trials have been reported to demonstrate the effects, or none of the diseases have advanced to Phase III after completion of the trials. Thus, no antibody drugs are recognized as being close to market launch at present.

Although, as described above, clinical trials have also been conducted for Crohn's disease and inflammatory bowel disease, which are diseases of interest in the present invention, these are Phase II trials examining the safety in which effects on Crohn's disease and inflammatory bowel disease have not been demonstrated. Therefore, it is necessary to wait for future reports on confirmation of the efficacy. However, it is not expected that the antibody drugs will be put into practical use. This is because, although the clinical trials for inflammatory bowel disease ended in 2012, and reports on safety and the like have been made, no clinical trial has been conducted in Phase III.

### Solution to Problem

The present invention relates to a therapeutic agent for inflammatory bowel disease, and in particular, to a therapeutic agent for intractable inflammatory bowel disease resistant to existing drugs.
(1) A pharmaceutical composition for treatment of inflammatory bowel disease, comprising, as an active ingredient, an anti-active IL-18 neutralizing antibody, or Fab, Fab', F(ab')₂, a single chain antibody (scFv), a disulfide-stabilized V region fragment (dsFv), a low-molecular-weight antibody (Fv-clasp) or a peptide containing CDR of the anti-active IL-18 neutralizing antibody.
(2) The pharmaceutical composition for treatment of inflammatory bowel disease according to (1), further comprising a TNF-α inhibitor as an active ingredient.
(3) The pharmaceutical composition for treatment of inflammatory bowel disease according to (1), which is administered in combination with a pharmaceutical composition containing a TNF-α inhibitor as an active ingredient.
(4) The pharmaceutical composition for treatment of inflammatory bowel disease according to (2) or (3), wherein the TNF-α inhibitor is an anti-TNF-α antibody, or Fab, Fab', F(ab')₂, a single chain antibody (scFv), a disulfide-stabilized V region fragment (dsFv), a low-molecular-weight antibody (Fv-clasp) or a peptide containing CDR of the anti-TNF-α antibody.
(5) A pharmaceutical composition for treatment of inflammatory bowel disease, comprising, as an active ingredient, a bispecific antibody comprising a first antigen-binding site that binds to an N-terminal region of active IL-18 and a second antigen-binding site that binds to TNF-α, or Fab, Fab', F(ab')₂, a single chain antibody (scFv), a disulfide-stabilized V region fragment (dsFv), a low-molecular-weight antibody (Fv-clasp) or a peptide containing CDR of the bispecific antibody.
(6) The pharmaceutical composition for treatment of inflammatory bowel disease according to any one of (1) to (5), wherein the inflammatory bowel disease is ulcerative colitis or Crohn's disease.
(7) The pharmaceutical composition for treatment of inflammatory bowel disease according to any one of (1) to (6), which is for inflammatory bowel disease resistant to existing treatment.
(8) The pharmaceutical composition for treatment of inflammatory bowel disease according to any one of (1) to (7), comprising a peptide in which a heavy chain variable domain of the anti-active IL-18 neutralizing antibody or the first antigen-binding site that binds to an N-terminal region of active IL-18 comprises a CDR1H region consisting of the amino acid sequence of GFSLSSSGMG (SEQ ID NO: 1), a CDR2H region consisting of the amino acid sequence of IWWDDDK (SEQ ID NO: 2), and a CDR3H region consisting of the amino acid sequence of TRTRTYSNFGGGMAY (SEQ ID NO: 3), and a light chain variable domain comprises a CDR1L region consisting of the amino acid sequence of QSIAHSNGYTY (SEQ ID NO: 4), a CDR2L region consisting of the amino acid sequence of KVS (SEQ ID NO: 5), and a CDR3L region consisting of the amino acid sequence of VQGSHVPLT (SEQ ID NO: 6); or a peptide in which the heavy chain variable domain comprises a CDR1H region consisting of the amino acid sequence of GFSLTSYG (SEQ ID NO: 7), a CDR2H region consisting of the amino acid sequence of IWAGGST (SEQ ID NO: 8), and a CDR3H region consisting of the amino acid sequence of ARESSYDAMDY (SEQ ID NO: 9), and the light chain variable domain comprises a CDR1L region consisting of the amino acid sequence of ENVVTY (SEQ ID NO: 10), a CDR2L region consisting of the amino acid sequence of GAS (SEQ ID NO: 11), and a CDR3L region consisting of the amino acid sequence of GQGYSYPYT (SEQ ID NO: 12).
(9) The pharmaceutical composition for treatment of inflammatory bowel disease according to any one of (1) to (7), wherein an amino acid sequence of a H-chain variable region of the anti-active IL-18 neutralizing antibody or the first antigen-binding site that binds to the N-terminal region of active IL-18 is SEQ ID NO: 13, and an amino acid sequence of a L-chain variable region is SEQ ID NO: 14; or the amino acid sequence of the H-chain variable region is SEQ ID NO: 15, and the amino acid sequence of the L-chain variable region is SEQ ID NO: 16.
(10) A therapeutic effect prediction method for determining in advance whether a patient with inflammatory bowel disease as a subject has a therapeutic effect of the pharmaceutical composition for treatment of inflammatory bowel disease according to any one of (1) to (9), the method comprising measuring the expression levels of active IL-18 in the subject.
(11) A method for treating inflammatory bowel disease, comprising treating a patient with inflammatory bowel disease using the pharmaceutical composition according to (1), (2), or (5).
(12) A method for treating inflammatory bowel disease, comprising administering to a patient with inflammatory bowel disease the pharmaceutical composition according to (1) in combination with a pharmaceutical composition comprising, as an active ingredient, an anti-TNF-α antibody, or Fab, Fab', F(ab')₂, a single chain antibody (scFv), a disulfide-stabilized V region fragment (dsFv), a low-molecular-weight antibody (Fv-clasp) or a peptide containing CDR of the anti-TNF-α antibody.
(13) A method for treating inflammatory bowel disease, comprising confirming in advance efficacy of treatment with an anti-active IL-18 neutralizing antibody by the method according to (10) and providing treatment by the method according to (11) or (12).

### Brief Description of Drawings

[Figure 1A] Figure 1A shows a higher concentration of IL-18 in patients with Crohn's disease who are resistant to existing treatment.
[Figure 1B] Figure 1B shows immunostaining images by anti-active IL-18 antibody of endoscopic specimens from patients with Crohn's disease who are resistant to existing treatment.
[Figure 2] Figure 2 shows sequence homology of IL-18 between human and mouse.
[Figure 3A] Figure 3A shows results of examining epitopes of a 5-4.1 antibody recognizing active mouse IL-18 by alanine scanning method.
[Figure 3B] Figure 3B shows results of examining epitopes of a 9-3.1 antibody recognizing active mouse IL-18 by alanine scanning method.
[Figure 4A] Figure 4A shows an experimental schedule for an induction of enteritis by dextran sulfate sodium (DSS) and antibody administration.
[Figure 4B] Figure 4B shows effects of a 5-4.1 antibody, an anti-active IL-18 neutralizing antibody, on DSS-induced enteritis as analyzed by body weight change.
[Figure 4C] Figure 4C shows effects of an anti-active IL-18 neutralizing antibody on DSS-induced enteritis as analyzed by disease activity index score.
[Figure 5A] Figure 5A shows results of examining the mechanism of enteritis suppression by administration of an anti-active IL-18 neutralizing antibody based on the concentration of orally administered FITC-dextran in the serum.
[Figure 5B] Figure 5B shows results of analyzing the mechanism of enteritis suppression by administration of an anti-active IL-18 neutralizing antibody using tissue staining of constituent molecules of tight junctions.
[Figure 5C] Figure 5C shows results of analyzing the mechanism of enteritis suppression by administration of an anti-active IL-18 neutralizing antibody, using PAS staining of goblet cells secreting intestinal mucus. The results of counting the number of goblet cells are shown on the right.
[Figure 6A] Figure 6A shows that the administration of an anti-active IL-18 neutralizing antibody in combination with an anti-TNF-α antibody significantly ameliorates DSS-induced enteritis as indicated by percent weight loss.
[Figure 6B] Figure 6B shows that the administration of an anti-active IL-18 neutralizing antibody in combination with an anti-TNF-α antibody significantly ameliorates DSS-induced enteritis as indicated by disease activity index score.
[Figure 6C] Figure 6C shows that the administration of an anti-active IL-18 neutralizing antibody in combination with an anti-TNF-α antibody significantly ameliorates DSS-induced enteritis based on intestinal length.
[Figure 6D] Figure 6D shows HE-stained images of large intestine specimens revealing that administration of an anti-active IL-18 neutralizing antibody in combination with an anti-TNF-α antibody significantly ameliorates DSS-induced enteritis.
[Figure 7A] Figure 7A shows an experimental schedule for an induction of enteritis by 2,4,6-trinitrobenzenesulfonic acid (TNBS) and antibody administration.
[Figure 7B] Figure 7B shows effects of an anti-active IL-18 neutralizing antibody on TNBS-induced enteritis as analyzed by percent weight loss.
[Figure 7C] Figure 7C shows effects of an anti-active IL-18 neutralizing antibody on TNBS-induced enteritis as analyzed by disease activity index score.
[Figure 7D] Figure 7D shows effects of an anti-active IL-18 neutralizing antibody on TNBS-induced enteritis as analyzed by intestinal length.
[Figure 8A] Figure 8A shows that the administration of an anti-active IL-18 neutralizing antibody in combination with an anti-TNF-α antibody significantly ameliorates TNBS-induced enteritis, as indicated by percent weight loss.
[Figure 8B] Figure 8B shows that the administration of an anti-active IL-18 neutralizing antibody in combination with an anti-TNF-α antibody significantly ameliorates TNBS-induced enteritis as indicated by disease activity index score.
[Figure 8C] Figure 8C shows that the administration of an anti-active IL-18 neutralizing antibody in combination with an anti-TNF-α antibody significantly ameliorates TNBS-induced enteritis based on intestinal length.
[Figure 9A] Figure 9A shows α diversity (left) and β diversity (right) of gut microbiota in the enteritis-uninduced group, the IgG-administered group, and the 5-4.1 antibody-administered group on day 9 after DSS administration.
[Figure 9B] Figure 9B shows the relative abundance of gut microbiota at the phylum level in the enteritis-uninduced group, the IgG-administered group, and the 5-4.1 antibody-administered group.
[Figure 9C] Figure 9C shows the relative abundance of gut microbiota at the genus level in the IgG-administered group and the 5-4.1 antibody-administered group, and the bacteria shown in the bar graph represent bacteria significantly increased in each group.
[Figure 9D] Figure 9D shows the relative abundance in each group of *Enterococcus, Staphylococcus,* and *Lactococcus,* whose relative abundance increased in the enteritis-induced group.
[Figure 10A] Figure 10A shows an epitope of an anti-IL-18 antibody as analyzed by Western blotting analysis.
[Figure 10B] Figure 10B shows an epitope of an anti-IL-18 antibody as analyzed by ELISA.
[Figure 10C] Figure 10C shows results of analyzing effects of a 5-4.1 antibody and an anti-IL-18 antibody on DSS-induced enteritis based on disease activity index score.
[Figure 10D] Figure 10D shows results of analyzing effects of a 5-4.1 antibody and an anti-IL-18 antibody on DSS-induced enteritis based on percent weight loss.
[Figure 11A] Figure 11A shows results of analyzing effects of a 5-4.1 antibody and an anti-IL-18 antibody on TNBS-induced enteritis based on disease activity index score.
[Figure 11B] Figure 11B shows results of analyzing effects of a 5-4.1 antibody and an anti-IL-18 antibody on TNBS-induced enteritis based on percent weight loss.

### Description of Embodiments

The present inventors have considered that one of the reasons why anti-IL-18 antibody medicaments are not readily applied clinically is that the antibodies used recognize not only active IL-18 but also abundant precursors. In view of this, the present inventors conducted an investigation using an antibody recognizing only active IL-18. Unlike cytokines such as TNF, IL-18 is not regulated for production at the mRNA level and is abundant in cells as an inactive precursor (pro-IL-18). The pro-IL-18 is released extracellularly in an active form when its precursor is cleaved by caspase-1 or caspase-4. Accordingly, the use of an antibody that binds to precursor protein does not necessarily lead to suppression of active IL-18. Moreover, even if the antibody binds to both the active form and the precursor, it does not efficiently suppress the activity of active IL-18 due to a large amount of the IL-18 precursor present in the cells.

The IL-18 antibody according to the present invention is an antibody recognizing only active IL-18, in which the epitope is a newly formed neoepitope resulting from cleavage of the precursor pro-IL-18 by caspase-1/4. The term "epitope" refers to a region of an antigen that is recognized by an antibody and means a site on the antigen to which a domain comprising an antibody variable region disclosed herein binds. For example, when a polypeptide such as IL-18 is used as an antigen, the epitope can be defined by the amino acid sequence or the structure of the antigen because the antibody may recognize a linear amino acid sequence or a three-dimensional structure. The neoepitope of IL-18 is a sequence present only in active IL-18 cleaved by caspase-1/4, and an antibody recognizing this can be an antibody recognizing only active IL-18.

The term antibody in the preset invention is intended to include a functional fragment of such an antibody that exhibit substantially the same antigen specificity as the original antibody, i.e., recognize the same epitope. The functional fragment of the antibody includes a functional fragment of an antibody such as Fab, Fab', F(ab')₂, a single chain antibody (scFv), a disulfide-stabilized V region fragment (dsFv), a low-molecular-weight antibody (Fv-clasp, Non Patent Literature 4) or a peptide containing CDR.

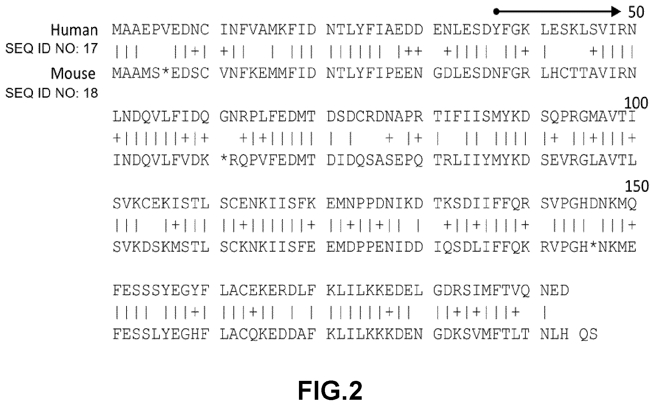

In Examples of the present invention, an antibody recognizing active IL-18 in mice is used because the N-terminal sequence of active IL-18 is different between human and mouse (see Figure 2), but it is needless to say that an antibody recognizing active human IL-18 needs to be used when applied to humans. Examples of such an antibody that recognizes active human IL-18 include a 9-10.2 antibody and an 8-4.1 antibody (Patent Literature 11) .

Specific examples thereof include an antibody (9-10.2 antibody) in which the heavy chain variable domain comprises a CDR1H region consisting of the amino acid sequence of GFSLSSSGMG (SEQ ID NO: 1), a CDR2H region consisting of the amino acid sequence of IWWDDDK (SEQ ID NO: 2), and a CDR3H region consisting of the amino acid sequence of TRTRTYSNFGGGMAY (SEQ ID NO: 3), and the light chain variable domain comprises a CDR1L region consisting of the amino acid sequence of QSIAHSNGYTY (SEQ ID NO: 4), a CDR2L region consisting of the amino acid sequence of KVS (SEQ ID NO: 5), and a CDR3L region consisting of the amino acid sequence of VQGSHVPLT (SEQ ID NO: 6); or an antibody (8-4.1 antibody) in which the heavy chain variable domain comprises a CDR1H region consisting of the amino acid sequence of GFSLTSYG (SEQ ID NO: 7), a CDR2H region consisting of the amino acid sequence of IWAGGST (SEQ ID NO: 8), and a CDR3H region consisting of the amino acid sequence of ARESSYDAMDY (SEQ ID NO: 9), and the light chain variable domain comprises a CDR1L region consisting of the amino acid sequence of ENVVTY (SEQ ID NO: 10), a CDR2L region consisting of the amino acid sequence of GAS (SEQ ID NO: 11), and a CDR3L region consisting of the amino acid sequence of GQGYSYPYT (SEQ ID NO: 12). Alternatively, the antibody that can be used is an antibody (9-10.2 antibody) in which the amino acid sequence of the H-chain variable region is SEQ ID NO: 13, and the amino acid sequence of the L-chain variable region is SEQ ID NO: 14; an antibody (8-4.1 antibody) in which the amino acid sequence of the H-chain variable region is SEQ ID NO: 15, and the amino acid sequence of the L-chain variable region is SEQ ID NO: 16; or an antibody having 80% or more, preferably 90% or more, more preferably 95% or more homology with these antibodies.

Furthermore, the 9-10.2 antibody and the 8-4.1 antibody, which recognize active human IL-18, are mouse monoclonal antibodies, but in the case of clinical application to humans, the antibodies of the present invention also include humanized antibodies obtained by converting these monoclonal antibodies into, for example, human chimeric antibodies and humanized CDR-grafted antibodies using gene recombination technology, and human antibodies using genetically modified mice. Humanized antibodies and human antibodies, when administered to humans, have fewer side effects than non-human animal antibodies and longer-lasting therapeutic effects. In addition, antibodies with stronger neutralizing activity can also be obtained by introducing mutations into the Fc region of IgG1 to reduce binding to C1q and Fc receptors, thereby preventing the antibodies from being taken up by immune cells such as macrophages. Such mutations include K322A, L234A, and L235A (Non Patent Literature 5), triple mutations of L234F/L235E/P331S (Non Patent Literature 6), and N297Q (Non Patent Literatures 7 and 8). Based on these findings, for example, an antibody (Patent Document 12) in which, for example, LALA mutations (L234A and L235A) are introduced to suppress uptake by immune cells such as macrophages, may be used.

As will be described in detail below, an investigation conducted using a mouse model has revealed that enteritis can be more strongly suppressed by using the antibody in combination with an anti-TNF-α antibody. When applied to humans, the anti-TNF-α antibody include, but are not limited to, existing antibody medicaments, such as Infliximab, adalimumab, golimumab, and certolizumab pegol. Anti-TNF-α antibody medicaments to be developed in the future can be used as well.

Furthermore, the use of an antibody against an IL-18 neoepitope in combination with an anti-TNF-α antibody remarkably produces the suppression effect, suggesting that any TNF inhibitor may be used. Examples thereof include an inhibitor that binds to TNF-α and inhibits its action, such as a soluble TNF-α receptor, and a low-molecular-weight compound that inhibits the action of TNF-α. In addition, a bispecific antibody that binds to active IL-18 and TNF-α, as well as a functional antibody fragment that maintains the antigen specificity of the bispecific antibody, can also be used as a pharmaceutical composition for treatment of inflammatory bowel disease.

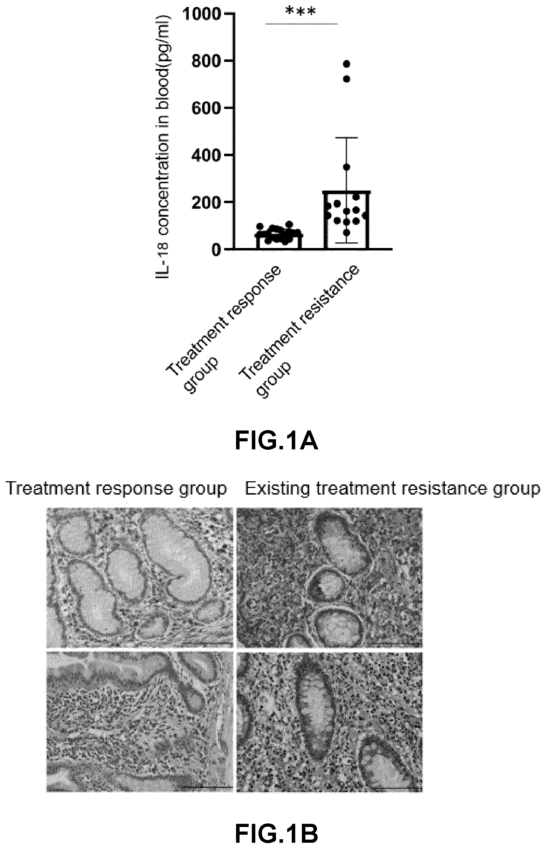

Hereinafter, the details will be described with data. In a group of patients with inflammatory bowel disease, differences between the treatment response group and the treatment resistance group were examined in response to an anti-TNF-α antibody and anti-IL-12/23 antibody preparations. As a result, it was revealed that IL-18 was significantly expressed at a higher level in the group resistant to the treatment (Figures 1A and 1B). IL-18 concentrations in the blood of the group of patients who responded to the treatment with the anti-TNF-α antibody and the anti-IL-12/23 antibody preparations and the group of patients who were resistant to the treatment were measured using a human IL-18 ELISA kit (MBL) (Figure 1A). The IL-18 concentration in the blood were significantly higher (*** indicates p < 0.001; the same applies hereafter) in the treatment resistance group than in the treatment response group.

Colonoscopy specimens from patients with Crohn's disease were immunostained using an antibody (9-10.2) that recognizes only active IL-18 (Figure 1B). Specimens obtained from patients in the group who are resistant to existing treatments showed higher expression of active IL-18 in the intestinal epithelium and stromal tissues than those from the treatment response group.

Given the higher concentration of IL-18 in the blood of the treatment resistance group and its presence as active IL-18 in large intestine specimens, an antibody that recognizes and neutralizes active IL-18 was used to examine whether it could suppress inflammatory bowel disease.

### [Production of antibody recognizing mouse IL-18 neoepitope]

In human inflammatory cytokine IL-18 protein (uniprot: Q14116, SEQ ID NO: 17), polypeptides at positions 37 to 193, which are cleaved by caspases, function as active IL-18. Although mouse IL-18 (NP_001344150, SEQ ID NO: 18) and human IL-18 are highly homologous, they differ in the sequence on the N-terminus that serves as the neoepitope of active IL-18 (in Figure 2, the C-terminal side from an arrow functions as active IL-18; the cleaved terminal serves as the neoepitope.). A mouse IL-18 neoepitope sequence (NFGRLHCTTC, SEQ ID NO: 19), in which cysteine (C) was added to the C-terminus of peptides at positions 36 to 44 of mouse IL-18 that were cleaved by caspases to form the neoepitope, was synthesized by a conventional method.

As a sensitizing antigen, keyhole-limpet hemocyanin (hereinafter referred to as KLH) was cross-linked to the peptide of SEQ ID NO: 19 using Imject Maleimide-Activated mcKLH spin Kit (Thermo Scientific) and then mice were immunized according to a conventional method.

The same peptide was cross-linked to bovine serum albumin (hereinafter referred to as BSA) using Imject Maleimide-Activated BSA spin Kit (Thermo Scientific) and screened by ELISA to select and establish hybridomas 5-4.1 and 9-3.1. Note that the antibody produced by hybridoma 5-4.1 is referred to as 5-4.1 antibody and the antibody produced by hybridoma 9-3.1 is referred to as 9-3.1 antibody.

Isotypes of monoclonal antibodies 5-4.1 and 9-3.1 that recognize active IL-18 produced by the hybridomas 5-4.1 and 9-3.1 were confirmed to be IgG1, κ using IsoStrip mouse monoclonal antibody isotyping kit (Sigma).

### [Examination of epitopes of 5-4.1 antibody and 9-3.1 antibody]

The 5-4.1 antibody and the 9-3.1 antibody were confirmed to be antibodies that recognize and neutralize only active mouse IL-18 by ELISA, immunoprecipitation, and mouse IL-18 functional inhibition activity method. Only the results of alanine scanning method by ELISA are shown below. When a protein is cleaved by a proteolytic enzyme such as a caspase as described above, protein terminal, neoepitopes, which are not present in a normal protein, are formed. Neoepitope peptides used for antibody production were subjected to epitope analysis by alanine scanning method. Here, quantitative analysis is performed by ELISA as described below.

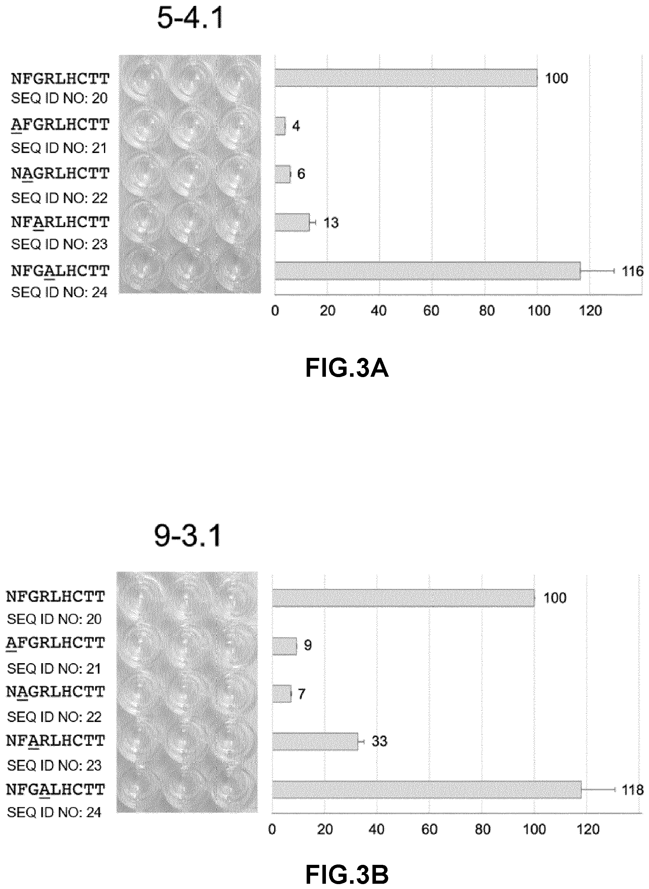

An N-terminal sequence peptide of an active mouse IL-18 protein (positions 36 to 44, NFGRLHCTT, SEQ ID NO: 20) and a peptide of alanine variants of each four amino acid of N-terminus (SEQ ID NOs: 21 to 24) in which biocytin was added to the C-terminus were synthesized according to a conventional method. Each peptide was immobilized on NeuroAvidin plates via biotin, and recognition sites of the 5-4.1 antibody and 9-3.1 antibody were analyzed by ELISA (Figures 3A and 3B). On the right of ELISA plate photograph in Figure 3, the absorbance obtained with a plate reader is shown together with the standard deviation based on the wild-type peptide as 100.

Both the 5-4.1 antibody and the 9-3.1 antibody recognized 10% or less of the first amino acid of the N-terminal sequence peptide (peptide in which asparagine (N) at position 36 was changed to alanine (A) (N36A)) and an F37A peptide of the active mouse IL-18 protein. They also showed slightly stronger reactivity to the R39A variant than to the wild type. On the other hand, the 5-4.1 antibody showed weak binding of about 10% of the wild-type peptide to the G38A variant, while the 9-3.1 antibody showed about 30% reactivity thereto.

The results of the alanine scanning method described above revealed that the peptides at positions 36 to 38 were especially important for recognition by the 5-4.1 antibody and the 9-3.1 antibody. In other words, it was revealed that both the 5-4.1 antibody and the 9-3.1 antibody recognized NFGRLHCTT (SEQ ID NO: 20) as an epitope and NFG (SEQ ID NO: 25) as a minimum epitope. Next, the 5-4.1 antibody was investigated to determine whether it had a therapeutic effect on two mouse models with inflammatory bowel disease (mouse models with DSS-induced enteritis and TNBS-induced enteritis).

### [Example 1]

### [Therapeutic effect of 5-4.1 antibody on mouse model with DSS-induced enteritis]

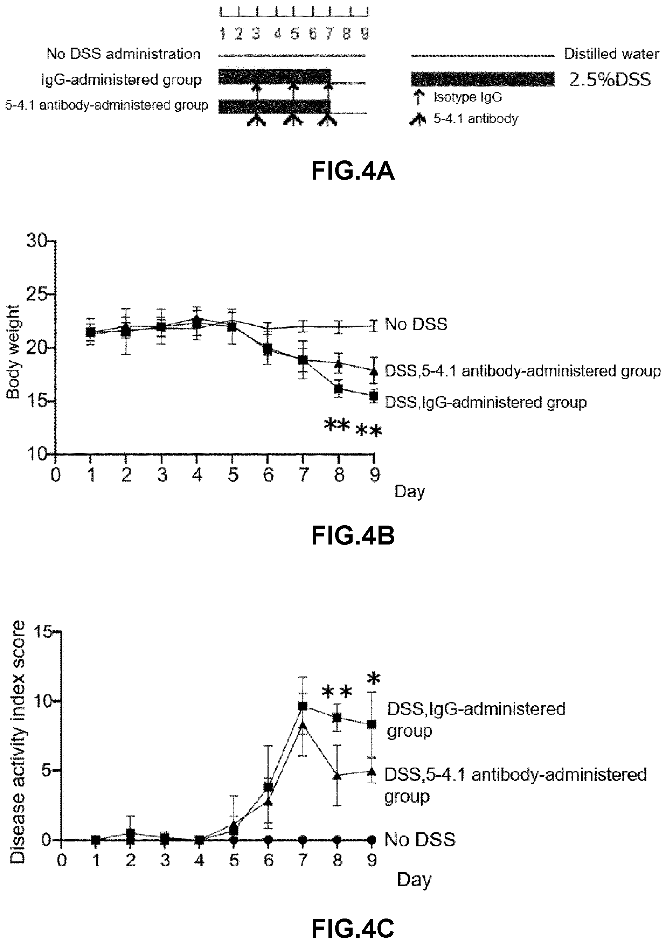

DSS (dextran sulfate sodium)-induced enteritis is a model of acute enteritis that induces DSS-induced intestinal epithelial cell damage and activation of macrophages, Th1 cells and Th17 cells. Eight-week-old C57BL/6J male mice were divided into two groups: an enteritis-induced group fed with drinking water containing 2.5% DSS to induce enteritis and an uninduced group fed with distilled water. The enteritis-induced group was further divided into a 5-4.1 antibody-administered group and an isotype IgG (InVivoMab mouse IgG1 isotype control BioXCell)-administered group (control group), which received DSS for 7 days (Figure 4A). The experiment was conducted with 6 mice in each group. The 5-4.1 antibody or isotype IgG was intraperitoneally administered at a dose of 200 µg each on days 3, 5 and 7 after the start of DSS administration, and the mice were dissected on day 9. The disease activity index score determined from body weight (Figure 4B), stool shape, degree of hematochezia and percent weight loss (Non Patent Literature 9, Figure 4C) was measured daily. Compared with the control group, the 5-4.1 antibody-administered group showed significantly reduced body weight loss and improved disease activity index score (** indicates p < 0.01, and * indicates p < 0.05; the same applies hereafter). When dissection was performed on day 9 after the start of DSS administration, the 5-4.1 antibody-administered group showed suppression of intestinal length shortening and improvement in pathological score (Non Patent Literature 9), which scored inflammatory cell infiltration into the submucosal layer and epithelial damage. Based on the results above, the 5-4.1 antibody was identified to ameliorate enteritis in a mouse model with DSS-induced enteritis.

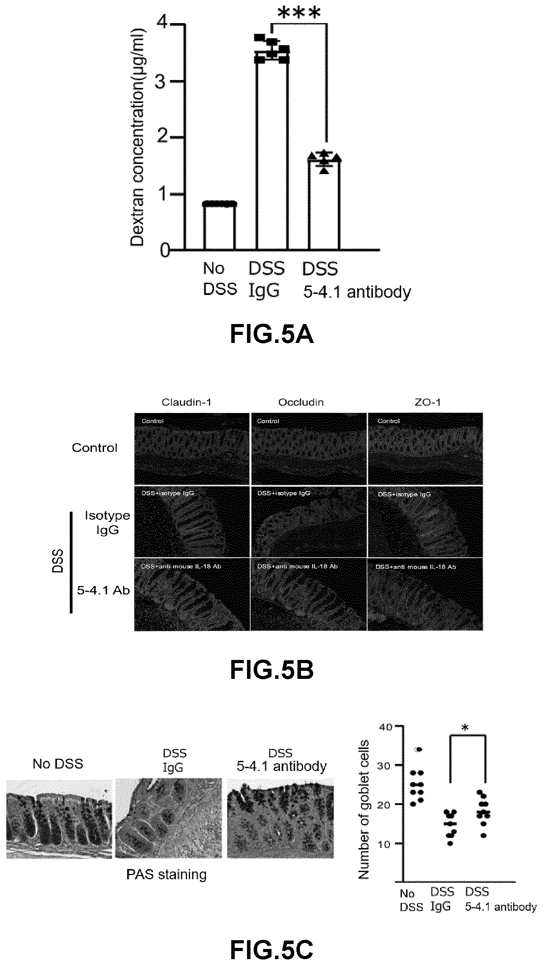

A possible mechanism by which DSS induces enteritis include a disruption of the defense mechanism of intestinal epithelial cells, excessive activation of immune cells and dysbiosis. To verify the effect of the 5-4.1 antibody on the intestinal epithelium, mice were fed with DSS for 7 days, 4 kDa dextran labeled with FITC was orally administered to the mice on day 9 after the start of induction of enteritis, and blood was collected 4 hours later to measure the concentration of FITC-dextran in the serum (Figure 5A). On day 9 after the induction of enteritis, the permeability of dextran increased, and an increase in the concentration of dextran in blood was observed. In the 5-4.1 antibody-administered group, however, the concentration of FITC-dextran in blood was decreased as compared with the control group, and an improvement in increased permeability was observed.

Immunohistochemical staining of claudin-1, occludin and ZO-1, molecules constituting tight junctions that regulate permeability, was performed (Figure 5B). Large intestine tissue specimens obtained from a DSS-free group (control group), an IgG-administered group in which enteritis was induced by DSS administration and to which isotype IgG was further administered, or the 5-4.1 antibody-administered group were subjected to immunohistochemical staining by a conventional method. The primary antibodies used were an anti-claudin-1 antibody (Invitrogen, #71-7800), an anti-occludin antibody (Invitrogen, #40-4700), and an anti-ZO-1 antibody (#61-7300, Thermo Scientific). The administration of DSS decreased the expression of all of the above molecules constituting tight junctions (DSS + Isotype IgG), while the administration of the 5-4.1 antibody (DSS + 5-4.1 Ab) improved the decreased expression of claudin-1 and occludin. In addition, the large intestine tissue specimens were subjected to PAS staining to observe goblet cells secreting intestinal mucus. The number of goblet cells was significantly increased in the 5-4.1 antibody-administered group compared with the isotype IgG-administered group (Figure 5C, right graph).

These results indicated that the 5-4.1 antibody restored the expression of the molecules constituting tight junctions, which were reduced by DSS administration, suppressed the increased permeability, and protected the function of intestinal epithelium through restoration of the number of goblet cells. Although data are not shown here, the 5-4.1 antibody-administered group showed decreased expression of inflammatory cytokines IFN-γ and TNF-α and a chemokine CXCL2 as compared with the control group.

### [Example 2]

### [Therapeutic effect of 5-4.1 antibody used in combination with anti-TNF-α antibody on DSS-induced enteritis]

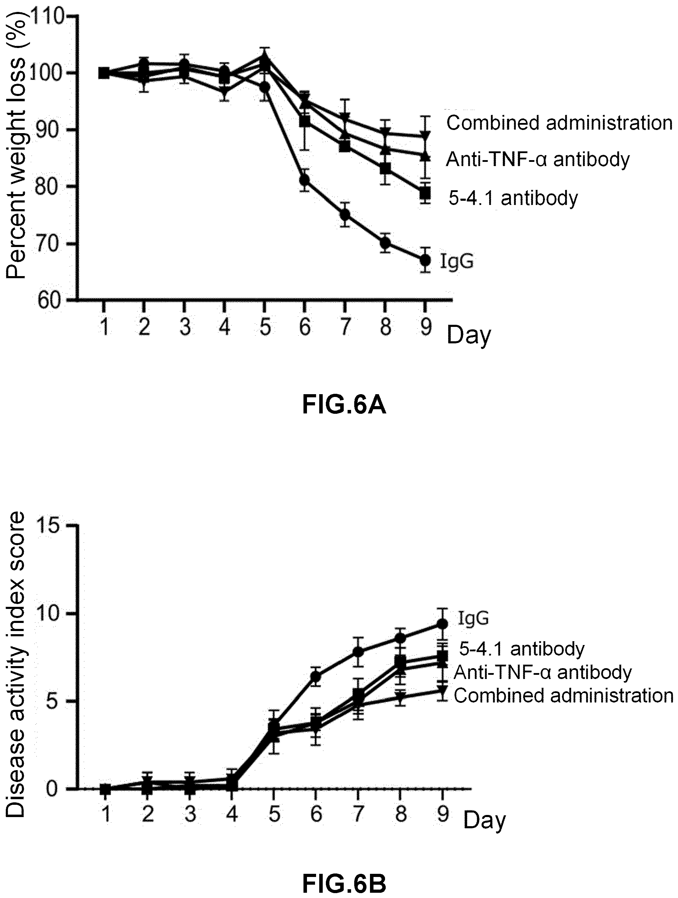

Anti-TNF-α antibodies have been clinically applied as therapeutic agents for IBD. To investigate the use of an anti-TNF-α antibody in combination with an anti-active IL-18 antibody which was found to be effective, therapeutic effects of an anti-mouse TNF-α antibody (Invivo Mab anti-mouse TNF-α, BioXCell) used in combination with the 5-4.1 antibody on DSS-induced enteritis were verified. The mice were divided into four groups: an isotype IgG-administered group (control group), a 5-4.1 antibody-administered group, an anti-TNF-α antibody-administered group, and a combined administration group of the 5-4.1 antibody and the anti-TNF-α antibody. Enteritis was induced by the same protocol to verify the therapeutic effects (Figures 6A to 6D). Enteritis was induced by DSS in the same manner as in Example 1, and the 5-4.1 antibody and the anti-TNF-α antibody were each intraperitoneally administered at a dose of 100 µg. In the combined administration group, 100 µg of the 5-4.1 antibody and 100 µg of the anti-TNF-α antibody were intraperitoneally administered, while 100 µg of isotype IgG was intraperitoneally administered as a control, on days 3, 5, and 7 from the start of DSS administration for all the groups.

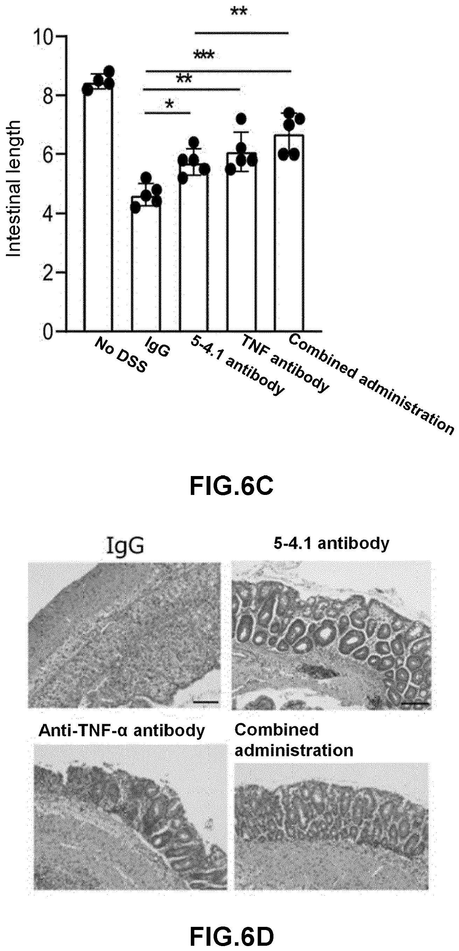

Compared with the anti-TNF-α antibody-administered group, the 5-4.1 antibody-administered group showed a similar amelioration effect on enteritis in terms of percent weight loss and disease activity index score, and the use of the 5-4.1 antibody in combination with the anti-TNF-α antibody showed a remarkable effect of ameliorating enteritis in terms of both percent weight loss and disease activity index score (Figures 6A and 6B). The 5-4.1 antibody-administered group also showed a similar effect of improvement in the intestinal length shortening caused by inflammatory enteritis as the anti-TNF-α antibody-administered group, while the combined administration group showed the stronger effect of improvement than the group of single administration (Figure 6C). The 5-4.1 antibody-administered group also showed a similar effect of improvement in pathological examination as the anti-TNF-α antibody-administered group, while the combined administration group showed the stronger effect of improvement than the group of single administration (Figure 6D). Although the results are not shown here, the production of anti-TNF-α was more significantly suppressed in the combined administration group than in the 5-4.1 antibody-administered group.

### [Example 3]

### [Therapeutic effect of 5-4.1 antibody on mouse model with TNBS-induced enteritis]

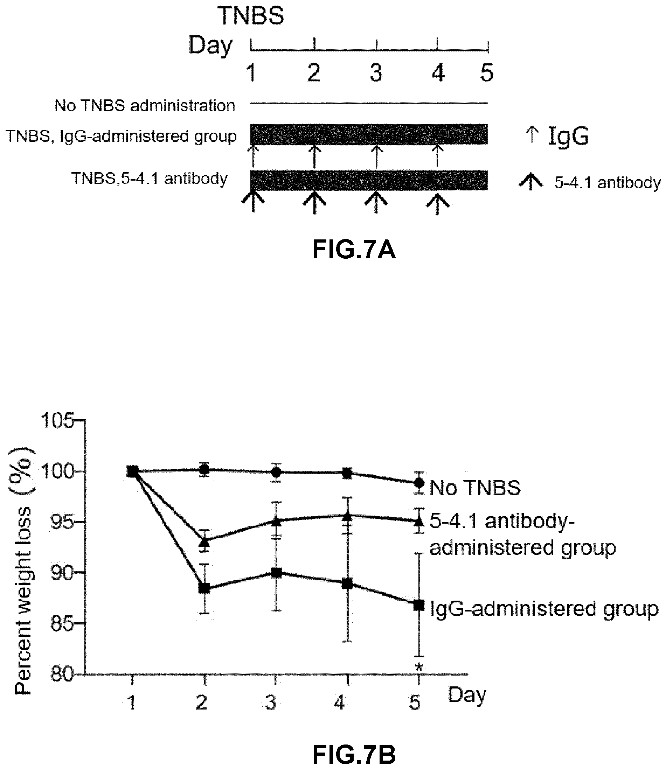

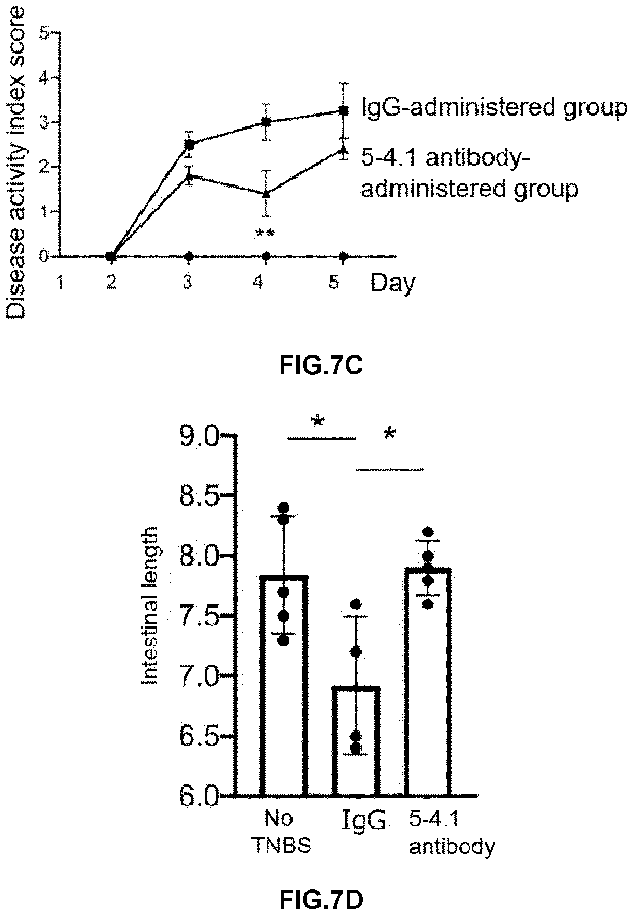

TNBS (2,4,6-trinitrobenzenesulfonic acid)-induced enteritis, a model of acute enteritis in which transrectal administration of TNBS induces activation of Th1 cells, is regarded as a model of Crohn's disease. As shown in Figure 7A, the transrectal administration of TNBS (10 mg/ml, 100 µl) was performed on day 1, the 5-4.1 antibody or control IgG was each intraperitoneally administered at a dose of 100 µg on days 1, 2, 3 and 4, and the mice were dissected on day 5. Compared with the control group, the 5-4.1 antibody-administered group showed significantly reduced percent weight loss (Figure 7B) and improved disease activity index score (Figure 7C). When dissection was performed, the 5-4.1 antibody-administered group showed suppression of intestinal length shortening (Figure 7D), as well as pathological improvement. Based on the results above, the 5-4.1 antibody was identified to ameliorate enteritis in a mouse model with TNBS-induced enteritis.

### [Example 4]

### [Therapeutic effect of 5-4.1 antibody used in combination with anti-TNF-α antibody on TNBS-induced enteritis]

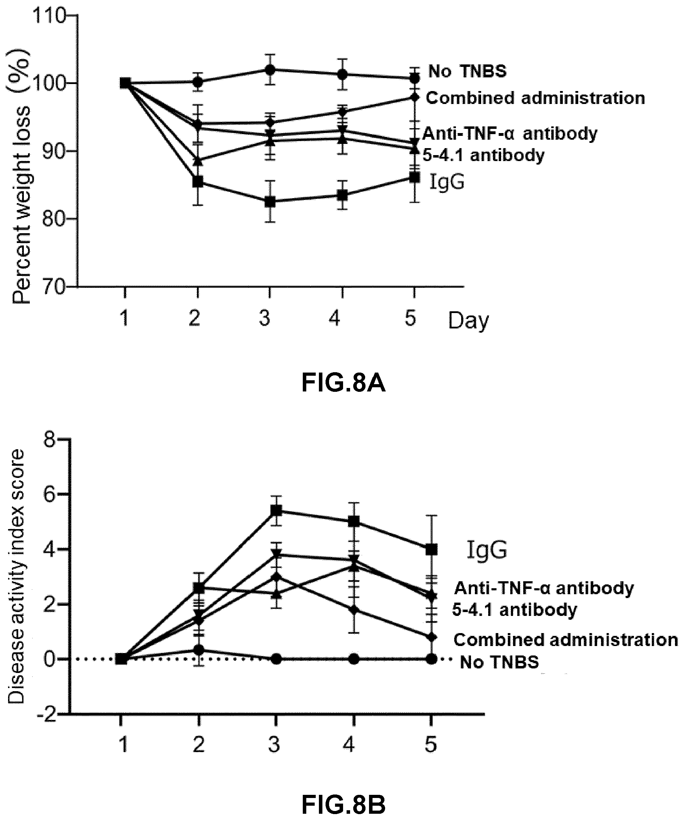

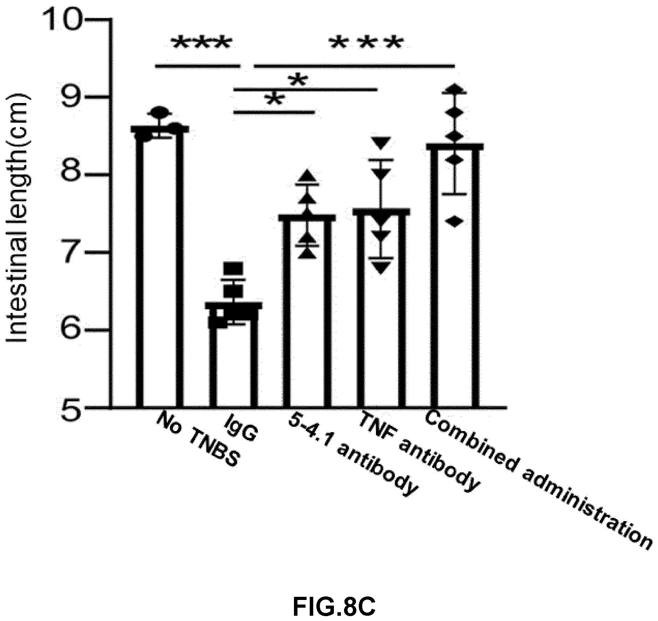

The therapeutic effects of the anti-TNF-α antibody used in combination with the anti-5-4.1 antibody on TNBS-induced enteritis were verified (Figure 8). The mice were divided into four groups: an IgG-administered group (control group), a 5-4.1 antibody-administered group, an anti-TNF-α antibody-administered group and a combined administration group of the 5-4.1 antibody and the anti-TNF-α antibody. Enteritis was induced by the same protocol as in Example 3 to verify the therapeutic effects. Specifically, TNBS was administered on day 1, and the 5-4.1 antibody and the anti-TNF-α antibody were intraperitoneally administered alone or in combination at a dose of 100 µg each on days 1, 2, 3, and 4. Compared with the anti-TNF-α antibody-administered group, the 5-4.1 antibody-administered group showed a similar amelioration effect on enteritis in terms of percent weight loss (Figure 8A) and disease activity index score (Figure 8B), and the use of the 5-4.1 antibody in combination with the anti-TNF-α antibody showed a remarkable effect of ameliorating enteritis in terms of both percent weight loss and disease activity index score. Regarding the intestinal length, the 5-4.1 antibody-administered group also showed a similar effect of suppressing intestinal length shortening as compared with the anti-TNF-α antibody-administered group. The combined group had intestinal length similar to that of mice without TNBS treatment and showed significant effect (Figure 8C).

### [Example 5]

### [Effect of 5.4.1 antibody on gut microbiota]

As shown above, the 5-4.1 antibody-administered group showed therapeutic effects on both DSS-induced and TNBS-induced enteritis. Although the loss of diversity of gut microbiota has been known in inflammatory bowel disease, an investigation was conducted to determine whether administration of the anti-active IL-18 antibody could improve the intestinal environment.

In the same manner as in Example 1, 8-week-old C57BL/6J male mice were fed with DSS-containing drinking water for 7 days to induce enteritis. They were divided into 2 groups: a 5-4.1 antibody-administered groups and an isotype IgG-administered group (6 mice in each group), and a DSS-free group was used as a control. Stool samples were collected on day 9 after the initial administration of DSS to extract DNA from the stool samples using DNeasy PowerSoil Kit (Qiagen). The V3-4 region of a 16SrRNA gene was amplified by 1st PCR and 2nd PCR to prepare a DNA library, and nucleotide sequence information was obtained using the next-generation sequencer MiSeq from Illumina, Inc. SILVA (Version 38) and QIIME2 were used to analyze the obtained nucleotide sequences. Linear discriminant analysis was used to compare the composition of the gut microbiota between groups. For analysis of α diversity and β diversity, evaluation was performed using Microbiome Analyst software 36.

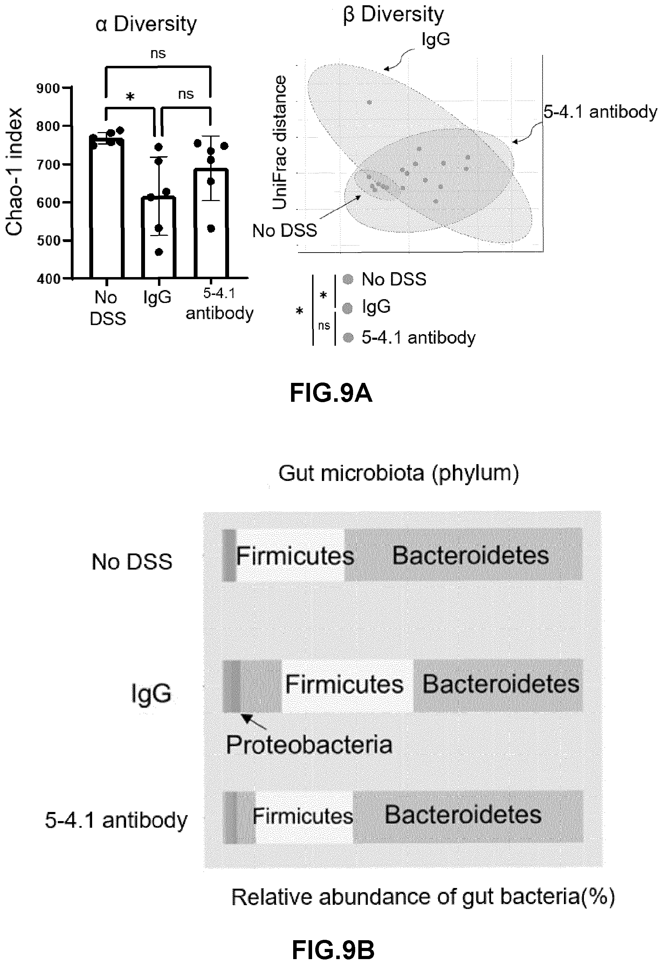

When the enteritis-uninduced group as a control, the IgG-administered group (enteritis-induced group) and the 5-4.1 antibody-administered group were compared, the Chao-1 index indicating α diversity was significantly decreased in the enteritis-induced group, but no significant difference was found between the IgG-administered group and the 5-4.1 antibody-administered group (Figure 9A, left). The UniFrac distance, which indicates β diversity, formed different clusters due to the induction of enteritis, but no significant difference was found between the IgG-administered group and the 5-4.1 antibody-administered group (Figure 9A, right).

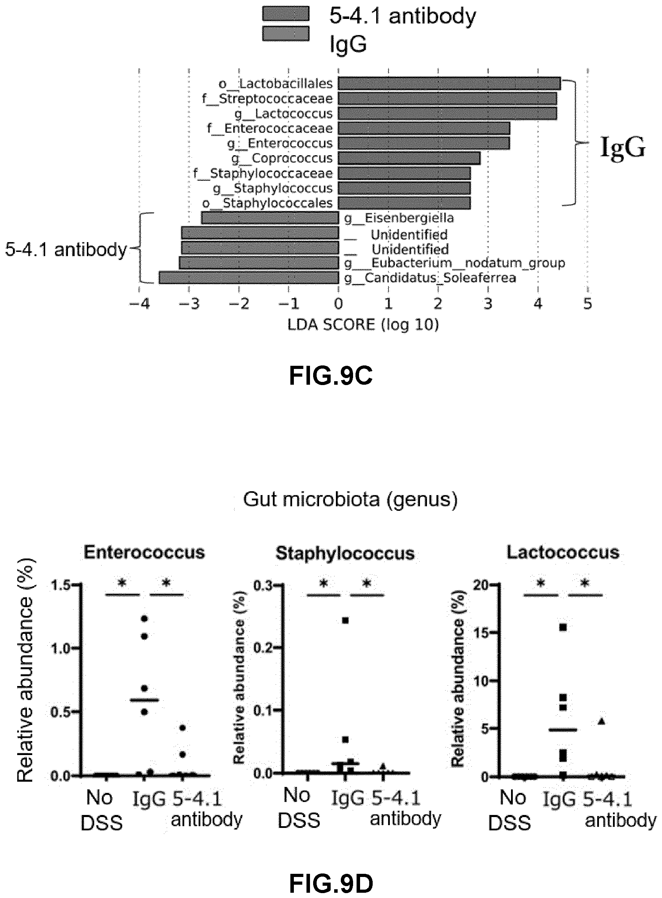

Regarding the composition of the microbiota, there were no significant differences at the phylum level, but there was a decrease in the relative abundances of Bacteroides phylum and an increase in the Firmicutes phylum in the enteritis-induced group. The 5-4.1 antibody-administered group showed a tendency to suppress these changes in the microbiota (Figure 9B). In addition, an analysis was performed at the genus level (Figure 9C). The relative abundances of the gut microbiota at the genus level in the IgG-administered group (enteritis-induced group) and the 5-4.1 antibody-administered group were analyzed to represent the bacteria that increased significantly at the genus level in each group. When the three groups were compared, the relative abundances of *Enterococcus, Staphylococcus* and *Lactococcus* increased in the IgG-administered group (enteritis-induced group), and the administration of 5-4.1 antibody significantly suppressed the increase (Figure 9D). Note that Tukey method was used to compare the three groups, and * indicates P < 0.05.

### [Example 6]

### [Comparison of enteritis suppression effect of 5-4.1 antibody and anti-mouse IL-18 antibody on mouse model with DSS-induced enteritis]

The effects of the antibodies on inflammatory enteritis shown in Examples were analyzed to determine whether they were specific to the 5-4.1 antibodies, antibodies that recognize neoepitopes, or whether the effects could be achieved if antibodies had IL-18 neutralizing activity. An anti-mouse IL-18 antibody (Bio X cell, BE0237) (hereafter referred to as an anti-IL-18 antibody) is known to have neutralizing activity (Non Patent Literature 10). First, it was confirmed whether the anti-IL-18 antibody was a neutralizing antibody recognizing epitopes other than cleaved terminal (neoepitopes) by activation of IL-18.

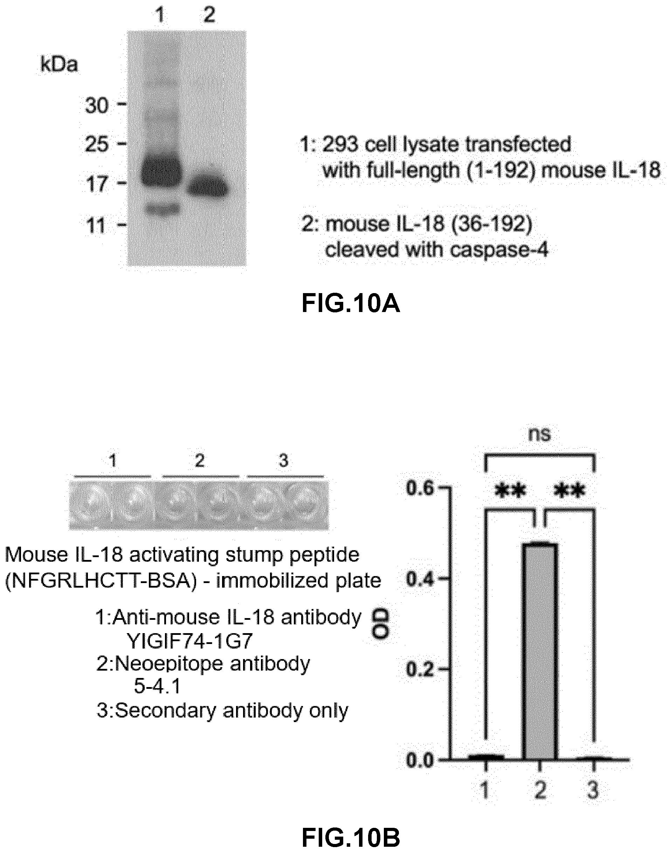

Full-length mouse IL-18 or active mouse IL-18 (positions 36 to 192) proteins were prepared and subjected to Western blotting analysis. Note that active mouse IL-18 was purified as follows: Full-length mouse IL-18 expressed in *E. coli* was purified and then mixed with active caspase-4¹⁰⁵⁻³⁷⁷ (which means peptides at positions 105 to 377 of caspase-4) expressed and purified in *E. coli* to purify and obtain active mouse IL-18 proteins (IL-18³⁶⁻¹⁹²). The N-terminal sequence of the purified proteins determined by Edman degradation was determined, and then the N-terminal sequence was NFGR as expected. This corresponded to the N-terminus of active IL-18, asparagine at position 36 to arginine at position 39. Western blot analysis using these proteins revealed that the anti-IL-18 antibody recognized both full-length mouse IL-18 and active IL-18 (Figure 10A).

Furthermore, regions recognized by the anti-IL-18 antibody were confirmed by ELISA. The recognition sites (epitopes) of the anti-IL-18 antibody and the 5-4.1 antibody were analyzed by ELISA, in which BSA was cross-linked to a peptide (NFGRLHCTTC, SEQ ID NO: 19) obtained by adding cysteine for cross-linking to the C-terminus of the N-terminal sequence peptides (positions 36 to 44) of the active mouse IL-18 proteins using Imject Maleimide-Activated BSA spin Kit and immobilized on a plate (Figure 10B). The graph on the right shows each absorbance obtained with the plate reader with the mean ± standard deviation. ** indicates a P value < 0.005, and ns indicates no significant difference (P = 0.810). The anti-IL-18 antibody did not recognize any of the N-terminal sequence peptides, neoepitopes upon activation. These results demonstrated that the 5-4.1 antibody recognized the mouse IL-18 terminal peptide formed by activation, whereas the anti-IL-18 antibody did not recognize the mouse IL-18 terminal peptide formed by activation. In other words, the anti-IL-18 antibody is neutralizing antibody recognizing regions other than the neoepitopes.

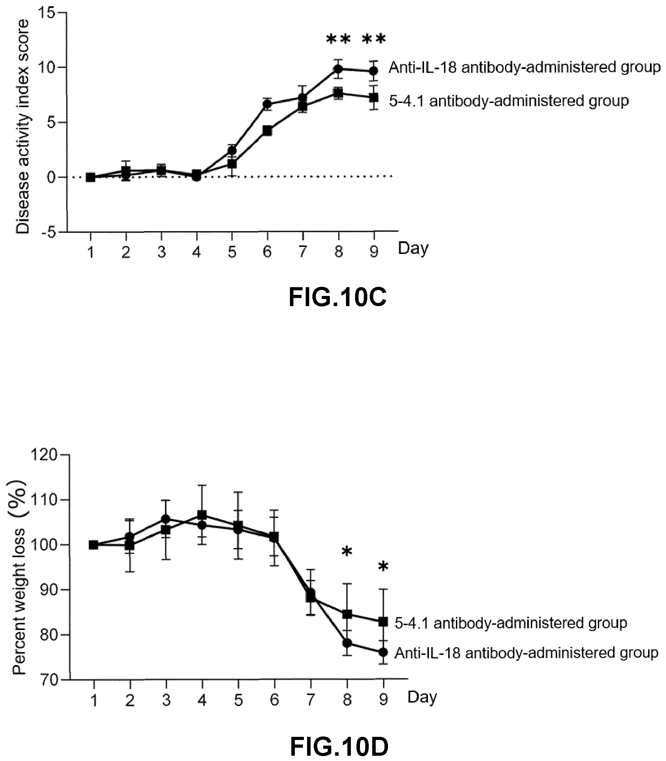

Eight-week-old C57BL/6J male mice were divided into two groups: a 5-4.1 antibody-administered group and an anti-mouse IL-18 antibody-administered group (5 mice in each group), which received drinking water containing 2.5% DSS for 5 days. The 5-4.1 antibody or anti-mouse IL-18 antibody was intraperitoneally administered at a dose of 200 µg on days 3, 5, and 7, and the mice were dissected on day 9. The disease activity index score (Figure 10C) and body weight (Figure 10D) determined from stool shape, degree of hematochezia, and percent weight loss were measured daily. Compared with the anti-mouse IL-18 antibody-administered group, the 5-4.1 antibody-administered group showed significantly reduced body weight loss and improved disease activity index score (** indicates p < 0.01, and * indicates p < 0.05.). The above results indicated that the 5-4.1 antibody significantly ameliorated enteritis in a mouse model with DSS-induced enteritis as compared with the anti-mouse IL-18 antibody.

### [Example 7]

### [Comparison of enteritis suppression effect of 5-4.1 antibody and anti-mouse IL-18 antibody on mouse model with TNBS-induced enteritis]

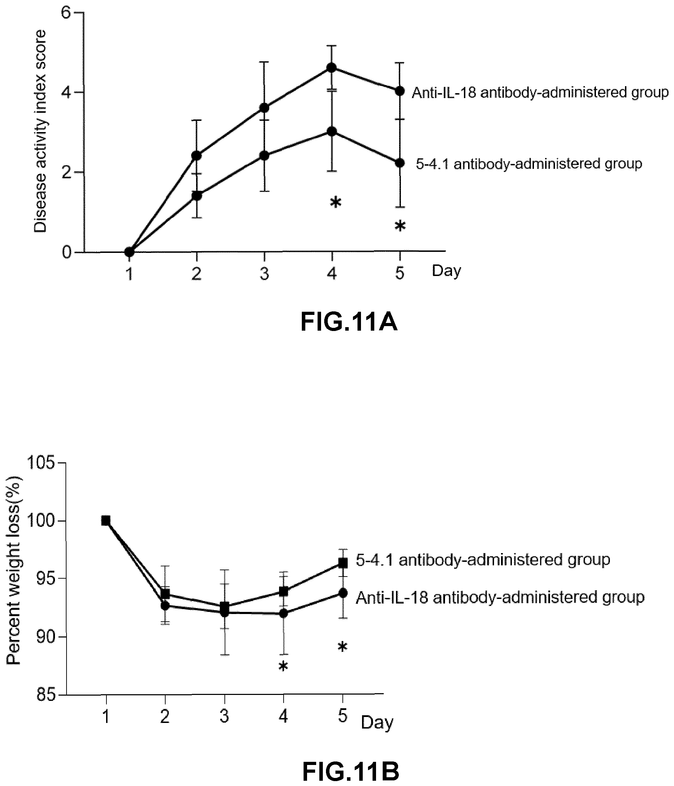

A mouse model with TNBS-induced enteritis was also analyzed to determine whether the therapeutic effects of the 5-4.1 antibody and the anti-mouse IL-18 antibody were different. Eight-week-old C57BL/6J male mice were subjected to transrectal administration of TNBS on day 1, and the 5-4.1 antibody or the anti-mouse IL-18 antibody was intraperitoneally administered at a dose of 100 µg each on days 1, 2, 3 and 4 (5 mice in each). The disease activity index score (Figure 11A) and body weight (Figure 11B) determined from stool shape, degree of hematochezia, and percent weight loss was measured daily (* indicates p < 0.05.). Compared with the anti-mouse IL-18 antibody-administered group, the 5-4.1 antibody-administered group showed significantly reduced body weight loss and improved disease activity index score. The above results indicated that the 5-4.1 antibody significantly ameliorated enteritis in a mouse model with TNBS-induced enteritis as compared with the anti-mouse IL-18 antibody.

As shown in Examples above, administration of the 5-4.1 antibody was found to ameliorate enteritis in two mouse models with IBD. In addition, the effect of the 5-4.1 antibody was significantly superior to that of an anti-IL-18 antibody binding to a site other than the neoepitope with neutralizing activity. Currently, there is no IL-18 suppression therapy leading to clinical application, and antibodies specific to active IL-18 could be novel therapeutic agents for intractable cases, which have become a major clinical problem. Since a more synergistic effect of ameliorating enteritis was observed in combination with an anti-TNF-α antibody as an existing treatment, a combination therapy of the anti-active IL-18 antibody and the anti-TNF-α antibody can be expected as a novel therapy for intractable cases.

The pathological conditions of IBD are diverse, and a plurality of factors are involved. Therefore, selection of treatments according to individual pathological conditions is required. The identification of a group of patients with high IL-18 expression will lead to identification of a group of patients in whom IL-18 is the main of pathological conditions and to therapeutic applications according to the pathological conditions. Specifically, the expression of IL-18, especially active IL-18, is confirmed at the stage of resistance to existing treatment. If high IL-18 expression is observed, treatment with an anti-active IL-18 antibody medicament or use of the anti-active IL-18 antibody medicament in combination with an anti-TNF-α antibody medicament may provide therapeutic effects. Furthermore, the expression of IL-18 is confirmed at the stage of resistance to treatment with 5-aminosalicylic acid preparations, immunomodulators such as azathioprine, and corticosteroids. Then, administration of anti-active IL-18 antibody medicaments when high IL-18 expression is observed may achieve higher effects.

## Claims

1. A pharmaceutical composition for treatment of inflammatory bowel disease, comprising, as an active ingredient, an anti-active IL-18 neutralizing antibody, or Fab, Fab', F(ab')₂, a single chain antibody (scFv), a disulfide-stabilized V region fragment (dsFv), a low-molecular-weight antibody (Fv-clasp) or a peptide containing CDR of the anti-active IL-18 neutralizing antibody.

2. The pharmaceutical composition for treatment of inflammatory bowel disease according to claim 1, further comprising a TNF-α inhibitor as an active ingredient.

3. The pharmaceutical composition for treatment of inflammatory bowel disease according to claim 1, which is administered in combination with a pharmaceutical composition containing a TNF-α inhibitor as an active ingredient.

4. The pharmaceutical composition for treatment of inflammatory bowel disease according to claim 2 or 3, wherein the TNF-α inhibitor is an anti-TNF-α antibody, or Fab, Fab', F(ab')₂, a single chain antibody (scFv), a disulfide-stabilized V region fragment (dsFv), a low-molecular-weight antibody (Fv-clasp) or a peptide containing CDR of the anti-TNF-α antibody.

5. A pharmaceutical composition for treatment of inflammatory bowel disease, comprising, as an active ingredient, a bispecific antibody comprising a first antigen-binding site that binds to an N-terminal region of active IL-18 and a second antigen-binding site that binds to TNF-α, or Fab, Fab', F(ab')₂, a single chain antibody (scFv), a disulfide-stabilized V region fragment (dsFv), a low-molecular-weight antibody (Fv-clasp) or a peptide containing CDR of the bispecific antibody.

6. The pharmaceutical composition for treatment of inflammatory bowel disease according to any one of claims 1 to 5, wherein the inflammatory bowel disease is ulcerative colitis or Crohn's disease.

7. The pharmaceutical composition for treatment of inflammatory bowel disease according to any one of claims 1 to 6, which is for inflammatory bowel disease resistant to existing treatment.

8. The pharmaceutical composition for treatment of inflammatory bowel disease according to any one of claims 1 to 7, comprising a peptide in which a heavy chain variable domain of the anti-active IL-18 neutralizing antibody or the first antigen-binding site that binds to an N-terminal region of active IL-18 comprises a CDR1H region consisting of the amino acid sequence of GFSLSSSGMG (SEQ ID NO: 1), a CDR2H region consisting of the amino acid sequence of IWWDDDK (SEQ ID NO: 2), and a CDR3H region consisting of the amino acid sequence of TRTRTYSNFGGGMAY (SEQ ID NO: 3), and a light chain variable domain comprises a CDR1L region consisting of the amino acid sequence of QSIAHSNGYTY (SEQ ID NO: 4), a CDR2L region consisting of the amino acid sequence of KVS (SEQ ID NO: 5), and a CDR3L region consisting of the amino acid sequence of VQGSHVPLT (SEQ ID NO: 6); or a peptide in which the heavy chain variable domain comprises a CDR1H region consisting of the amino acid sequence of GFSLTSYG (SEQ ID NO: 7), a CDR2H region consisting of the amino acid sequence of IWAGGST (SEQ ID NO: 8), and a CDR3H region consisting of the amino acid sequence of ARESSYDAMDY (SEQ ID NO: 9), and the light chain variable domain comprises a CDR1L region consisting of the amino acid sequence of ENVVTY (SEQ ID NO: 10), a CDR2L region consisting of the amino acid sequence of GAS (SEQ ID NO: 11), and a CDR3L region consisting of the amino acid sequence of GQGYSYPYT (SEQ ID NO: 12).

9. The pharmaceutical composition for treatment of inflammatory bowel disease according to any one of claims 1 to 7, wherein an amino acid sequence of a H-chain variable region of the anti-active IL-18 neutralizing antibody or the first antigen-binding site that binds to the N-terminal region of active IL-18 is SEQ ID NO: 13, and an amino acid sequence of a L-chain variable region is SEQ ID NO: 14; or the amino acid sequence of the H-chain variable region is SEQ ID NO: 15, and the amino acid sequence of the L-chain variable region is SEQ ID NO: 16.

10. A therapeutic effect prediction method for determining in advance whether a patient with inflammatory bowel disease as a subject has a therapeutic effect of the pharmaceutical composition for treatment of inflammatory bowel disease according to any one of claims 1 to 9, the method comprising measuring the expression levels of active IL-18 in the subject.
